# FASCICULE DE BREVET EUROPEEN

(11) **EP 2 303 243 B1**
(45) Date de publication et mention de la délivrance du brevet: **01.03.2017**
(21) Numéro de dépôt: 09766010.4
(22) Date de dépôt: 15.06.2009
(51) Int. Cl.: A61K 9/48, A61K 31/4365, A61K 47/30, A61P 7/02

(54) **COMPOSITION ORALE CONTENANT DU CLOPIDOGREL SOUS FORME DE BASE**
ORALE ZUSAMMENSETZUNG MIT CLOPIDOGREL ALS BASE
ORAL COMPOSITION CONTAINING CLOPIDOGREL IN ITS BASE FORM

(30) Priorité: 16.06.2008 FR 0803358
(43) Date de publication de la demande: 06.04.2011
(73) Titulaire: CLL Pharma, 06299 Nice cedex 3 (FR)
(72) Inventeur: BREUL, Thierry, F-34110 Frontignan (FR); LARUELLE, Claude, 06270 Villeneuve-Loubet (FR)
(74) Mandataire: Cabinet Armengaud Aîné
(86) Numéro de dépôt international: PCT/FR2009/000707
(87) Numéro de publication internationale: WO 2009/153448

(56) Documents cités:
- EP-A- 1 621 200
- WO-A-2004/074215
- WO-A-2007/024472
- WO-A-2008/134601
- WO-A1-2008/134600
- WO-A2-2007/137040
- WO-A2-2008/060934
- FR-A- 2 782 455
- US-A1- 2003 215 496
- DATABASE WPI Week 199546 Thomson Scientific, London, GB; AN 1995-355195 XP002509908 -& JP 07 242568 A (EISAI CO LTD) 19 septembre 1995 (1995-09-19)

## Description

L'invention se rapporte à des formulations orales contenant un agent anti-plaquettaire de la famille des thiénopyridines sous forme de base, le clopidogrel sous forme de base, à un procédé de préparation de celles-ci, ainsi qu'à leur utilisation dans la prévention de pathologies liées à une artérothrombose.

L'athérosclérose est la cause la plus fréquente des maladies thrombotiques cardio- et cérébro-vasculaires, et la cause éventuelle d'une morbidité et d'une mortalité prématurées à partir de la quarantaine. Selon la population étudiée et la méthode utilisée, la prévalence des pathologies artérielles est estimée entre 25 et 60%, et celle de l'infarctus du myocarde à 2% (Touze E et al.. Arch Mal Coeur Vaiss. 2005;98(4):15-30.

Parmi les traitements proposés dans la prévention des évènements liés à la l'athérothrombose (infarctus du myocarde, accident vasculaire cérébral, mort vasculaire), on trouve les agents anti-plaquettaires appartenant à la famille des thiénopyridines comme la ticlopidine commercialisée sous le nom Ticlid®, et le clopidogrel comercialisé sous le nom de Plavix® ; ces 2 molécules étant décrites respectivement dans les brevets FR 7303503 pour la 1^{ère} molécule et EP 281459 pour la 2^{nde}. Une autre thiénopyridine plus récente, le prasugrel divulgué dans la demande JP06041139A, n'est pas encore commercialisée, mais a été montrée dans plusieurs études comme possédant une activité anti-plaquettaire (Wiviott SD et al., 2005, Circulation, 111(25) :3366-73).

La spécialité Ticlid® se trouve sous forme de sel de chlorhydrate et la spécialité Plavix® sous forme de sel d'hydrogénosulfate. Aucune formulation administrable par voie orale du clopidogrel ou de ticlopidine sous forme de base n'est commercialisée.

Les brevets EP 099802, EP 281459 et US 4,847,265 décrivent des compositions à base de clopidogrel ou d'un de ses sels pharmaceutiquement acceptables à administrer par voie parentérale et se présentant sous forme d'un soluté injectable. Il s'avère que ce type de formulation qui contient un sel du clopidogrel en solution dans un solvant isotonique ne convient pas à une administration par voie orale, parce que le clopidogrel se dégrade par une réaction d'oxydation en présence d'eau, et parce que les compositions obtenues ne peuvent être conservées sous forme stable au delà d'un mois. On peut ajouter que ce type de solution dont le pH est inférieur à 2, provoquerait probablement, dans le cas d'une administration par voie orale, des irritations au niveau de l'estomac à cause de son pH très acide.

La demande de brevet WO 97/17064 décrit une forme lyophilisée pour administration parentérale ou par sonde ; elle contient outre le clopidogrel ou l'un de ses sels, du mannitol et de l'alanine.

La demande de brevet WO 00/10534 décrit une composition pharmaceutique aqueuse injectable à base de clopidogrel ou de ticlopidine ; cette composition contenant en outre un agent basique comme le phosphate disodique, et les 2 tensioactifs Pluronic® F680 (Poloxamer 188) et Solutol® HS15 ; le premier étant utilisé comme inhibiteur d'agrégation. Le principe actif est utilisé de préférence sous forme de sel, c'est d'ailleurs sous cette forme qu'il est exemplifié. A partir d'un lyophilisat, on reconstitue une solution dans un solvant de reprise aqueux qui est alors une solution micellaire du clopidogrel ayant un pH supérieur à 3, ce qui aurait pour avantage selon les Inventeurs de s'affranchir du problème d'auto-agrégation du clopidogrel grâce à l'utilisation des deux agents tensioactifs. Cependant la composition décrite nécessite, avant son administration, une reconstitution extemporanée par dissolution du lyophilisat afin de limiter la dégradation du clopidogrel en présence d'eau. D'après cet art antérieur, on constate que la préparation d'une forme liquide à base d'un sel pharmaceutiquement acceptable du clopidogrel ou de la ticlopidine s'avère très difficile à cause des propriétés physico-chimiques de ces principes actifs et notamment de leur instabilité chimique en présence d'eau.

Les demandes WO 04/026879A et WO 06/044548A décrivent des compositions contenant du clopidogrel sous forme de base ; la première décrit une forme amorphe de clopidogrel, ainsi qu'un procédé de préparation d'un complexe par co-dissolution du clopidogrel base (soit directement sous cette forme, soit générée *in situ* à partir de l'addition d'un acide dans une solution de clopidogrel bisulfate) et d'un polymère de N-vinyl pyrrolidone suivie par une étape d'évaporation du solvant pour former un résidu solide sec. La demande WO 06/044548 décrit un « prémix » (ou prémélange) solide à base de clopidogrel base qui est adsorbé sur un excipient choisi parmi un sucre, un disaccharide et un dérivé cellulosique. La forme galénique obtenue selon cette invention est un comprimé, une poudre, un sachet et apparentés ; l'objectif étant selon les Inventeurs d'améliorer la fluidité du clopidogrel base, de permettre aussi une meilleure homogénéité et de faciliter la préparation du « prémix ». Selon les Inventeurs, ce type de formulation et de préparation permettent d'améliorer la stabilité, la solubilité, la mise en oeuvre galénique et de réduire l'hygroscopicité. Cependant, ces 2 demandes PCT ne font nullement référence à une réduction des effets indésirables tel que l'hémolyse et l'irritation gastrique liés à l'emploi de ces agents anti-plaquettaires.

La demande de brevet WO2008/060934 décrit une composition pharmaceutique sous forme d'émulsion huile dans eau, dont la phase huileuse comprend un agent anti-plaquettaire, entre autre le clopidogrel sous forme de base.

L'administration du Ticlid® ou du Plavix® provoque fréquemment des effets indésirables. Parmi ceux-ci, et comme déjà évoqué, on peux citer les irritations, voire des saignements, au niveau stomacal. En solution aqueuse, ils donnent des solutions présentant un pH inférieur à 2, ce qui est incompatible avec une bonne tolérance gastrique lors de l'administration par voie orale. Ainsi, il est mentionné dans la notice du Ticlid® la recommandation : « à prendre au cours des repas pour diminuer les troubles digestifs ». Pour le Plavix®, la tolérance a été étudiée chez plus de 42000 patients dont plus de 9000 ont été traités pendant un an ou plus. Les effets indésirables notables au plan clinique qui ont été enregistrés au cours de l'étude CAPRIE, incluent notamment, chez les malades qui ont reçu du clopidogrel, une fréquence de survenue de saignements gastro-intestinaux de 2,0% ayant nécessité une hospitalisation dans 0,7% des cas, l'administration de ticlopidine est même contre indiquée pour les patients atteints d'ulcère gastroduodénal.

Parmi les autres effets indésirables liés à l'administration de ticlopidine ou de clopidogrel, on trouve aussi les effets hémorragiques ou atteintes hématologiques, dont certaines sont liées à l'hémolyse.

Dans le cas du Ticlid®, et dans la majorité des cas, ces accidents surviennent pendant les 3 premiers mois de traitement, et une surveillance biologique (prise de sang) est nécessaire pendant cette période (tous les 15 jours en général). Les cas répertoriés de toxicité hématologique (sur les cellules du sang) sont la chute du taux de globules blancs, de plaquettes, de globules rouges (anémie) ou des trois lignées de cellules, ce qui est gravissime. Dans ce cas, toute modification de la numération des cellules sanguines dans ce sens impose l'arrêt du traitement. De même, les hémorragies digestives, ORL, ophtalmologiques, cérébrales et méningées imposent l'arrêt du traitement.

Parmi les atteintes hématologiques répertoriées après administration du clopidogrel, on relève la microangiopathie thrombotique (MAT) qui désigne une lésion de l'endothélium des artérioles et des capillaires, cette lésion est commune à deux syndromes cliniques qui associent thrombopénie et anémie hémolytique. L'anémie hémolytique est considérée comme une anémie sévère, l'hémolyse est intravasculaire et le caractère microangiopathique de cette anémie est objectivé par la présence de nombreuses déformations des globules rouges. Des cas de MAT ont été rapportés après administration de ticlopidine ou de clopidogrel, causant le syndrome hémolytique urémique (SHU) : (Anderson F. et al Heart 90 : 9, 57, 2004 ; Medina PJ et al, Curr. Opin. Hematol 8 : 5, 286-93, 2001.).

Outre la MAT, on trouve comme pathologie hémolytique le Favisme, qui correspond à une déficience en G6PD (glucose-6-phosphate déshydrogénase) qui est une enzyme des globules rouges. Il a été démontré qu'une déficience de cette enzyme, provoque une hémolyse ou destruction de ces derniers conduisant à une anémie. Cette déficience en G6PD est très courante chez certaines catégories de population, par exemple, elle affecte plus de 10% des afro-américains et est particulièrement répandue chez les peuples du bassin méditerranéen, se manifestant par des crises d'hémolyse aigüe. Le déficit en G6PD est le plus commun des déficits en enzymes chez l'homme, on estime à plus de 400 millions le nombre de personnes affectées par un déficit en G6PD.

D'après ce qui a été exposé précédemment, il s'avère qu'il existe un besoin pour développer une formulation à base d'anti-plaquettaire comme le clopidogrel ou la ticlopidine, qui permette de réduire notablement les effets indésirables comme l'hémolyse, l'irritation et/ou le saignement au niveau stomacal.

La Demanderesse a conçu un type de formulation ayant un fort potentiel pour réduire certains des effets indésirables évoqués précédemment, et en particulier l'hémolyse. Cette nouvelle composition permet d'obtenir un pH neutre après dilution dans l'eau. Enfin, cette nouvelle composition présente l'avantage de rendre l'absorption indépendante de la prise de nourriture, contrairement au clopidogrel sous forme de sel hydrogénosulfate pour lequel un effet repas (variations d'absorption en fonction de la prise ou non de nourriture) a été démontré lors d'une étude réalisée chez des volontaires sains à qui on a administré du clopidogrel sous forme de sel hydrogénosulfate (Nigori, RVS et al, Arzneimittel-Forschung, 2006, vol.56, n°11 pp.735-739).

L'invention est telle que définie dans les revendications. L'invention a pour objet une composition pharmaceutique orale non-hémolytique contenant un agent anti-plaquettaire de la famille des thiénopyridines, à un pH proche de la neutralité après dilution dans l'eau caractérisée en ce que cet agent anti-plaquettaire est le clopidogrel sous forme de base, et que cette formulation contient en outre au moins un tensioactif non-ionique tel que défini dans les revendications. On entend par l'expression « proche de la neutralité » un pH compris entre 5 et 8 après dilution dans l'eau. Les Inventeurs ont constaté que les nouvelles formulations s'affranchissaient du problème de solubilité que connaît le Plavix®, car celui-ci contient le clopidogrel sous forme de sel qui requiert un pH très faible pour être dissout ; ceci n'est plus nécessaire pour les formulations de l'invention. De plus, les nouvelles formulations ont l'avantage de permettre une dissolution du principe actif très similaire dans des milieux intestinaux simulés à jeun et nourri, ce qui évoque l'absence d'effet repas.

Parmi les agents anti-plaquettaires, on trouve le clopidogrel, la ticlopidine et le prasugrel ; le clopidogrel étant celui utilisé dans le cadre de l' invention.

Les Inventeurs ont en effet montré que dans le cas du clopidogrel l'utilisation de la base et non du sel hydrogénosulfate (Plavix®), en présence d'un certain type de tensioactif, ne permet non seulement de dissoudre la base, mais aussi de réduire des effets indésirables liés à l'utilisation du sel hydrogénosulfate du clopidogrel comme l'hémolyse ou l'acidité.

Dans le cadre de leurs travaux, les Inventeurs ont constaté que parmi la classe particulièrement fournie de tensioactifs commercialisés, certains appartenant à la classe des tensioactifs (TA) non-ioniques hydrophiles permettent une dissolution de la base qui est très faiblement hydrosoluble, et ceci dans des conditions appropriées. Outre cette propriété, les Inventeurs ont montré lors de tests *in vitro* que d'une façon surprenante, cette composition a la capacité de réduire de façon très avantageuse l'hémolyse comparé au Plavix®.

Les TA non-ioniques hydrophiles peuvent être choisis parmi les tensioactifs dont la balance hydrophile-lipophile est supérieure à 10, ou parmi un mélange de tensioactifs dont la balance hydrophile-lipophile dudit mélange est supérieure à 10 ; à cet égard on peut se référer au manuel "Handbook of Surfactants" par M.R. Porter, éditions Blackie & Son, 2nd Edition, 1994).

Selon l'invention les TA non-ioniques hydrophiles sont le Crémophor®EL, le Crémophor®RH-40, le Solutol® HS-15 ou le mélange du Crémophor®RH-40 et de la vitamineE/TPGS® et selon une disposition encore plus avantageuse, le Crémophor®RH-40 et le mélange Crémophor®RH-40 -vitamineE/TPGS®. Le Crémophor®EL et le Crémophor®RH-40 sont des émulsifiants vendus par BASF ; ils correspondent à des huiles de ricin polyoxyéthylénées ; le Solutol® HS-15 vendu par BASF correspond à des esters de polyoxyéthylène de l'acide 12-hydroxystéarique ; la vitamineE/TPGS® correspond au succinate du d-α-tocophéryl polyéthylène glycol 1000.

Dans le cas où la composition le requiert il peut s'avérer utile d'incorporer des conservateurs et/ou antioxydant comme par exemple le butyl-hydroxyanisole (BHA) butyl-hydroxytoluène (BHT), acide éthylène diamine tetracétique (EDTA) ou ses sels, les méthyl ou propylparabens, les sels des dérivés de l'acide para 10-hydroxybenzoïque.

Les Inventeurs ont par ailleurs montré d'une façon surprenante que, lors d'études *in vitro,* les nouvelles formulations permettent une dissolution du principe actif (étape préalable nécessaire à son absorption intestinale) insensible à l'effet repas. En effet, des tests réalisés dans des milieux intestinaux simulés qui reproduisent le bol intestinal à jeun (FaSSIF) et le bol intestinal nourri (FeSSIF) ne montrent aucune différence au niveau de la dissolution du principe actif entre ces 2 milieux. De ce fait, cela n'induit pas de différence entre les quantités de principe actif à disposition de la lumière intestinale pour absorption entre les états à jeun et nourri, contrairement à la spécialité Plavix® pour lequel les quantités de principe actif dissout sont différentes dans les milieux à jeun et nourri, induisant un effet repas tel que décrit dans la publication de Nigori et al présentée précédemment.

Selon une disposition particulière, la composition pharmaceutique selon l'invention telle que décrite précédemment se caractérise par une solubilité de l'agent anti-plaquettaire supérieure à 70 mg/ml dans un milieu intestinal selon la pharmacopée USP XXI à pH=7,5, à une température de 37°C.

Selon une autre disposition particulière, la composition pharmaceutique selon l'invention telle que décrite précédemment se caractérise par une dissolution de l'agent anti-plaquettaire supérieure à 95 % dans les milieux digestifs simulés FeSSIF ou FaSSIF en 30 minutes à une température de 37°C et sous agitation de 50 tours par minute.

L'invention a également pour objet une composition pharmaceutique telle que précédemment décrite sous la forme de gélule ; cette gélule contenant un dosage du clopidogrel ou de la ticlopidine compris entre 5 et 600 mg, encore plus avantageuse à un dosage de 300 mg qui correspond à une dose de charge dans le cas de patients souffrant d'un syndrome coronaire aigu, et la plus avantageuse de toutes à un dosage de 75 mg de clopidogrel qui correspond à celui du Plavix® commercialisé et prescrit à la plupart des patients atteints par une pathologie artérothrombotique.

La gélule selon l'invention contient de préférence, outre l'agent antiplaquettaire, au moins un tensioactif non ionique hydrophile. Selon une disposition avantageuse, la gélule selon l'invention contient 75 mg de clopidogrel base et entre 250 et 600 mg de tensioactif non-ionique hydrophile choisi parmi le Crémophor®RH-40, le Solutol® HS 15, le Crémophor® EL et un mélange de Crémophor® RH-40 et de VitamineE/TPGS. Selon une disposition encore plus avantageuse, la gélule selon l'invention contient 75 mg de clopidogrel base et 290 mg de Crémophor®RH-40 ou 75 mg d clopidogrel et 287 mg d Crémophor®RH-40 et de 3 mg de VitamineE/TPGS.

La gélule de l'invention sera préférentiellement constituée en gélatine molle ou dure ou en hydropropylméthylcellulose (HPMC). Elle peut être scellée ou banderollée.

D'une façon avantageuse, on peux utiliser une gélule de taille 1 ou de taille 0 ; celle de taille 1 étant préférée. En effet, l'administration orale de celle-ci, de taille plus petite, convient tout particulièrement aux personnes qui ont des difficultés à avaler les gélules de taille 0.

Les Inventeurs se sont, de plus, fixés pour but de fournir un procédé de solubilisation, qui soit simple à mettre en oeuvre et ne nécessite pas d'autres appareillages que ceux dont sont classiquement équipés les Laboratoires pharmaceutiques.

L'invention a également pour objet un procédé de solubilisation d'une composition pharmaceutique de l'invention, comprenant les étapes suivantes :
(a) au moins un tensioactif non-ionique hydrophile est préalablement chauffé à une température comprise entre 30°C et 60°C ;
(b) le principe actif est dissout sous agitation à une température comprise entre 30°C et 60°C dans au moins un tensioactif non-ionique hydrophile préalablement chauffé.

Dans le cadre de leurs travaux, les Inventeurs ont en effet constaté que préalablement à l'ajout du principe actif, il était préférable de chauffer le(s) tensioactif(s) de façon à diminuer leur viscosité et accélérer la dissolution du principe actif.

De plus, un autre objet de l'invention est un procédé de préparation de gélule telle que présentée précédemment, comprenant les étapes suivantes :
(a) au moins un tensioactif non-ionique hydrophile est préalablement chauffé à une température comprise entre 30°C et 60°C ;
(b) le principe actif est dissout sous agitation à une température comprise entre 30°C et 60°C dans au moins un tensioactif non-ionique hydrophile préalablement chauffé ;
(c) le mélange ainsi obtenu est éventuellement refroidi à température ambiante ;
(d) le mélange est éventuellement chauffé à une température comprise entre 30°C et 45°C ;
(e) le mélange est transféré dans les gélules.

Les Inventeurs ont voulu mettre au point une composition non-hémolytique contenant un agent anti-plaquettaire destinée à être utilisée dans la prévention de pathologies liées à l'artérothrombose.

Selon une disposition particulière de l'invention, ce type de formulation pourra être utilisé chez des patients traités pour des pathologies liées à l'arthérothrombose et qui ont subi auparavant des effets indésirables lors de l'utilisation de spécialités classiques contenant un agent anti-plaquettaire.

Selon une disposition avantageuse, les patients traités par la composition de l'invention sont ceux susceptibles de subir des effets indésirables liés à l'hémolyse, ou à l'acidité gastro-intestinale lorsque l'agent anti-plaquettaire comme le clopidogrel ou la ticlopidine est utilisé sous la forme de sel.

Selon une autre disposition avantageuse, les patients traités par la composition de l'invention sont choisis parmi ceux qui sont déficitaires en glucose-6-phosphate déshydrogénase (G6PG) les patients souffrant d'atteintes hématologiques comme la microangiopathie thrombotique, ou les patients souffrant du syndrome hémolytique urémique.

Selon une autre disposition avantageuse, les patients traités par la composition de l'invention sont des sujets souffrant d'ulcère gastroduodénal ou de saignements gastro-intestinaux.

L'invention sera mieux comprise à l'aide du complément de description qui suit, qui se réfère à des exemples de préparation de compositions non-hémolytiques de clopidogrel sous la forme de base, ainsi que des divers tests (hémolyse, solubilités, dissolution et pH) dans lesquels on compare les nouvelles formulations à la spécialité Plavix® ; et qui se réfère aux figures associées suivantes :
- la Figure 1 présente les résultats d'hémolyse (test AGL) sur le sang du témoin N°1 en comparant les formulations 1 à 4 de l'invention à la spécialité Plavix® ; toutes ayant une concentration de 10µg/ml en clopidogrel ;
- la Figure 2 correspond au même test que celui de la Figure 1 sur le sang du témoin N°2
- la Figure 3 correspond au même test que celui de la Figure 1 avec une concentration en clopidogrel de 16,7 µg/ml pour toutes les formulations y compris celle de Plavix® ;
- la Figure 4 correspond au même test que celui de la Figure 1, avec une concentration en clopidogrel de 16,7 µg/ml pour le Plavix® alors que celle des formulations 1 à 4 de l'invention a été augmentée à 200 µg/ml ;
- la Figure 5 représente un test de dissolution dans le milieu physiologique simulé FaSSIF comparant les formulations 5 à 8 de l'invention à la spécialité Plavix® ;
- la Figure 6 représente un test de dissolution dans le milieu physiologique simulé FeSSIF comparant les formulations 5 à 8 de l'invention à la spécialité Plavix®.

Il va de soi, toutefois, que ce complément de description n'est donné qu'à titre d'illustrations de l'invention et n'en constitue en aucune manière une limitation.

### EXEMPLE 1 : TEST D'HEMOLYSE

### COMPARAISON ENTRE LES FORMULATIONS SELON L'INVENTION ET LA SPECIALITE PLAVIX®

Afin de déterminer le potentiel hémolytique du clopidogrel, un test d'hémolyse a été conduit sur le Plavix® qui contient du clopidogrel sous forme de sel bisulfate ainsi que sur les formulations selon l'invention contenant du clopidogrel sous forme de base.

Les formulations 1 à 4 selon l'invention sont décrites dans le Tableau 1 suivant : elles contiennent chacune 75 mg de clopidogrel base et une quantité identique en tensioactif non ionique hydrophile utilisé; celui-ci étant différent dans chaque formulation.

Quant au comprimé pelliculé Plavix®; il est constitué de 97,87 mg sous forme clopidogrel hydrogénosulfate (75 mg en clopidogrel base), et des excipients suivants: lactose anhydre, amidon de maïs modifié, macrogol, cellulose microcristalline, huile de ricin hydrogénée, dioxyde de titane, oxyde ferrique rouge et cire de carnauba.

**Tableau 1 : Formulations sous forme de gélule de taille 0 selon l'invention**

| | Clopidogrel base (en mg) | Tensioactif non-ionique hydrophile (nom ; en mg) | | Quantité totale (en mg) |
|---|---|---|---|---|
| Formulation 1 | 75 | Crémophor®EL | 525 | 600 |
| Formulation 2 | 75 | Solutol®HS-15 | 525 | 600 |
| Formulation 3 | 75 | Crémophor® RH-40 | 525 | 600 |
| Formulation 4 | 75 | Crémophor® RH-40 | 519,75 | 600 |
| | | Vitamine E/TPGS | 5,25 | |

### Test d'hémolyse utilisé (AGLT)

Ce test d'hémolyse est le test de lyse au glycérol acidifié (Acidified Glycerol lysis test ou AGLT tel que décrit dans : A. Zanella et al dans British Journal of Haematology, 45 481-6, (1980). Il permet de mesurer in vitro la résistance osmotique des globules rouges en milieu acidifié, au moyen de la mesure de la densité optique (DO). La lyse cellulaire est déterminée en observant l'évolution de la turbidité qui intervient lorsque l'intégrité de la membrane plasmique est compromise. On considère qu'une hémolyse a lieu lorsque la DO observée après 30 minutes est inférieure ou égale à la moitié de la DO initiale (DO 50 atteinte).

Le test AGLT permet également de détecter des anémies hémolytiques héréditaires et auto-immunes (P.H.B. Bolton-Maggs et al « Guidelines for the diagnosis and management of hereditary spherocytosis » British Journal of Haematology, 126, 455-474 (2004)).

### Mode opératoire

- Réactif glycérolé : dilution de 1,1 ml de glycérol dans 15 ml de PBS et dans 50ml d'eau distillée ;
- tampon phosphate : dilution dans une ampoule de tampon phosphate Titrisol dans 500ml d'eau et pH ajusté à 6,85 ;
- tampon PBS : dilution de 8,1g de NaCl dans 900ml d'eau et dans 100ml de Tampon phosphate pH = 6.85.

- *Cuve témoin* : la cuve contient 1 ml de PBS et 2 ml de réactif glycérolé.
- *Cuve d'échantillon* : la solution de départ comprend 5 ml d'une des formulations 1 à 4 ou de Plavix® à laquelle on ajoute 20 µl de sang. Le temps d'incubation est de 30 minutes. Dans la cuve, on place 1 ml de la solution de départ à laquelle on ajoute 2 ml de réactif glycérolé.

Remarque: la concentration en clopidogrel la plus élevée est de 15µg/ml, ce qui correspond à 75mg (comprimé) dans 5 litres de sang. Cette concentration sera donc plus ou moins réduite lors de l'ajout du PBS (tampon phosphate isotonique).
- *Analyse* : On mesure la densité optique (DO) à 625 nm ; cette longueur d'onde donne la meilleure différence entre les cellules intactes et les cellules lysées.

### Tests réalisés :

### - à une concentration en clopidogrel base de 10 µg/ml

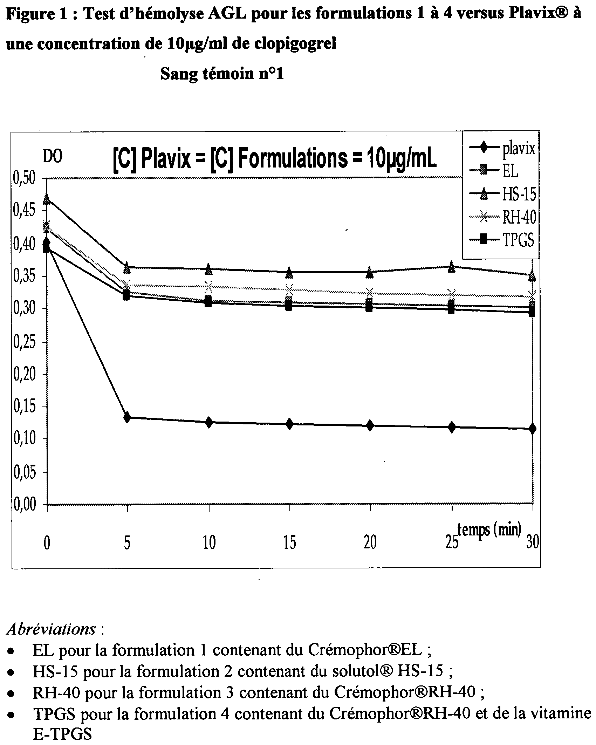

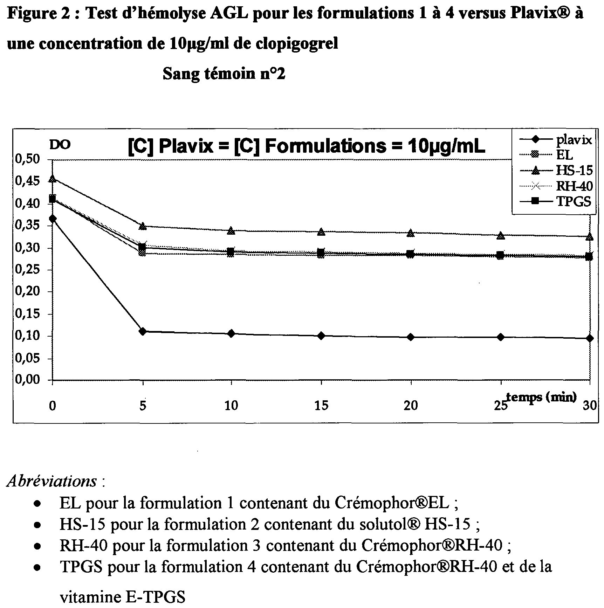

Le test a été réalisé sur 2 sangs de témoins sélectionnés différents. Pour chaque lot de sang de témoin sélectionné, une aliquote de 20µl est ajoutée à des solutions de Plavix® et des formulations 1 à 4 selon l'invention dans le PBS à une concentration de 10µg/ml. Chaque essai est réalisé en double. Les résultats présentés dans la figure 1 (sang témoin 1) et la figure 2 (sang témoin 2) sont la moyenne des deux essais.

Pour le sang du témoin N°1, on observe que la perte de DO est supérieure à 50% pour Plavix®, ce qui indique une hémolyse importante. En revanche, on observe une perte de DO réduite dans le cas des formulations 1 à 4 puisqu'elle est comprise entre 19% et 29%, ce qui indique une hémolyse très réduite, voire absente. L'expérience est tout fait répétable puisque les résultats obtenus sur le sang du témoin N°2 sont similaires : perte de DO supérieure à 50% pour Plavix®, et comprise entre 25% et 32% pour les formulations 1 à 4 de l'invention.

### - à une concentration en clopidogrel base de 16,7 µg/ml

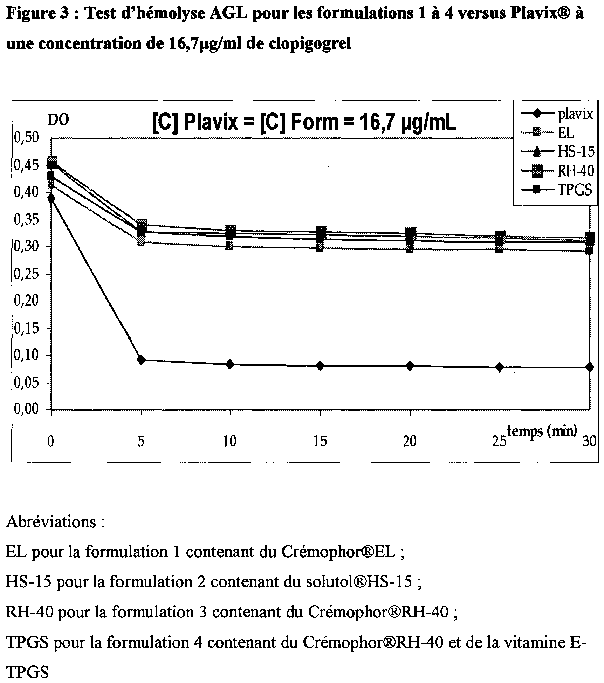

Une aliquote de 20 µl de sang du témoin sélectionné est ajoutée soit à une solution de Plavix®, soit à une des formulation 1 à 4 de l'invention dans du tampon PBS selon une concentration de 16,7µg/ml en clopidogrel. Chaque essai est réalisé en double. Les résultats sont présentés dans la figure 3. On constate que les résultats sont très semblables à ceux obtenus pour une concentration inférieure en clopidogrel (10 µg/ml). En effet, la perte en DO est supérieure à 50% pour Plavix®, ce qui implique une hémolyse importante. En revanche, la perte en DO est nettement réduite pour les formulations 1 à 4 (entre 29% et 32%) indiquant une hémolyse très réduite, voire absente.

### - à une concentration en clopidogrel base de 200 µg/ml pour les formulations 1 à 4

De façon à déterminer la dose en clopidogrel à partir de laquelle on observe une hémolyse importante pour les formulations de l'invention, il a été réalisé des tests à des concentrations de 100 µg/ml (résultats non montrés) et de 200 µg/ml de clopidogrel, tout en gardant une dose en clopidogrel nettement inférieure pour la solution contenant le Plavix® (16,7 µg/ml).

Comme décrit précédemment, on mélange une aliquote de 20 µl de sang d'un témoin et du tampon PBS à :
- soit à une solution de Plavix® à une concentration en clopidogrel de 16,7 µg/ml ;
- soit à l'une des formulations 1 à 4 de l'invention à une concentration en clopidogrel de 200 µg/ml

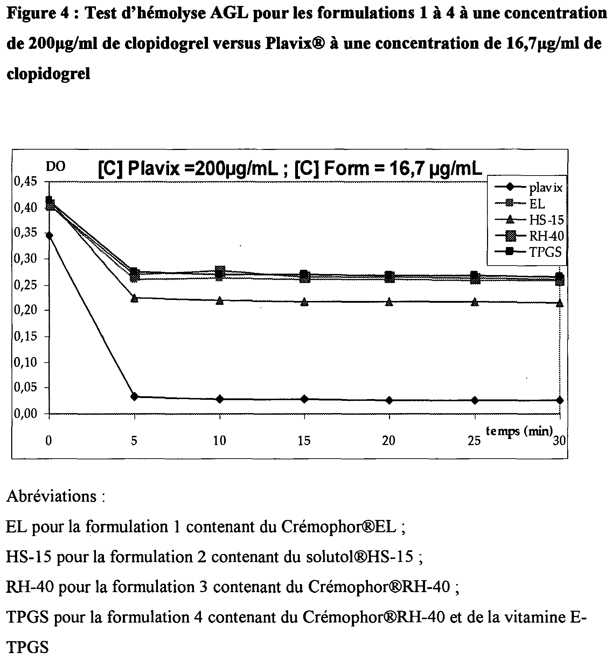

Chaque essai est réalisé en double. Les résultats sont présentés dans la figure 4. On constate qu'une hémolyse très importante a lieu pour le Plavix® (perte de DO supérieure à 50%) alors qu'elle est très réduite (perte DO comprise entre 29% et 47%) pour les formulations 1 à 4 selon l'invention, et ceci même pour des concentrations nettement plus élevées en clopidogrel (200 µg/ml).

### EXEMPLE 2 : TEST DE DISSOLUTION-

### COMPARAISON ENTRE LES FORMULATIONS SELON L'INVENTION ET LA SPECIALITE PLAVIX®

Dans cet exemple, la dissolution et la solubilité des formulations 5 à 8 (Tableau 2) de l'invention ont été évaluées par rapport à celles de la spécialité Plavix® dans les milieux intestinaux simulés FeSSIF et FaSSIF.

**Tableau 2 : Formulations 5 à 8 selon l'invention**

| | Clopidogrel base (en mg) | Tensioactif non-ionique hydrophile (nom ; en mg) | | Quantité totale (en mg) |
|---|---|---|---|---|
| Formulation 5 | 75 | Crémophor®EL | 290 | 365 |
| Formulation 6 | 75 | Solutol®HS-15 | 290 | 365 |
| Formulation 7 | 75 | Crémophor® RH-40 | 290 | 365 |
| Formulation 8 | 75 | Crémophor® RH-40 | 287 | 365 |
| | | Vitamine E/TPGS | 3 | |

Les milieux FaSSIF (Fasted Simulated Intestinal Fluid) et FeSSIF (Fed Simulated Intestinal Fluid) tels que décrits dans J.B. Dressman in Oral Drug Absorption, Drugs and the Pharmaceutical Sciences - Vol 106 . M. Dekker, 2000, simulent respectivement les fluides intestinaux à jeun et nourri.

*Mode opératoire* : Une quantité de 0,5 g de chaque formulation est introduite dans 1 ml de milieu intestinal simulé ; le mélange est placé à 37°C et est bien homogénéisé. Une dilution au 1/100^{ème} dans la phase mobile est effectuée avant d'être injectée dans une HPLC Perkin Elmer avec une colonne N°115. Pour le Plavix®, 3 comprimés sont introduits dans 1 ml de milieu.

### - Dissolution dans le milieu FaSSIF à 37°C

Le milieu FaSSIF est constitué de 3.9 g d'acide phosphorique monopotassique, 1.6 g de taurocholate de sodium, 0.6 g de lécithine, 7.7 g de chlorure de potassium ; qsp 11 avec de l'eau distillée. Le pH est ajusté à 6.5 avec de la soude

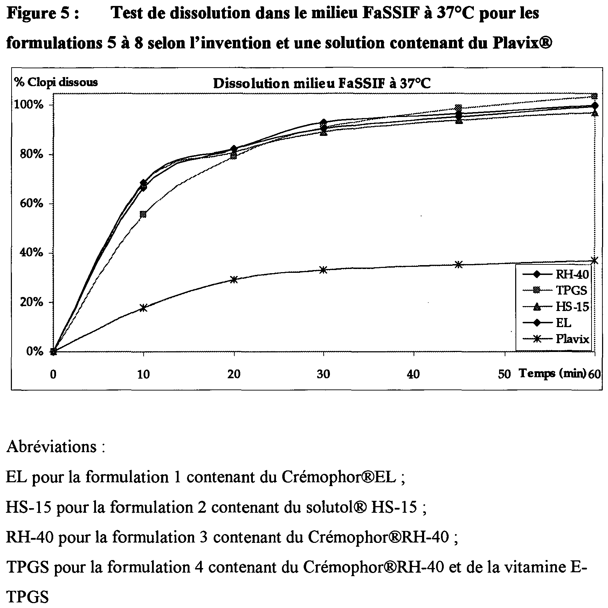

Les résultats sont présentés dans la Figure 5. On observe que seulement 37% du clopidogrel contenu dans Plavix® est dissous, alors presque la totalité (97%) l'est pour les formulations 5 à 8, et ceci après 30 minutes. Par ailleurs, on note que les 4 formulations de l'invention se comportent de façon très proche et que le choix du tensioactif non-ionique importe peu dans le cas du milieu FaSSIF.

### - Dissolution dans le milieu FeSSIF à 37°C

Le milieu FeSSIF est constitué de 8.7 g d'acide acétique, 8.1 g de taurocholate de sodium, 2.8 g de lécithine, 15.2 g de chlorure de potassium, qsp 11 avec de l'eau distillée. Le pH est ajusté à 5 avec de la soude

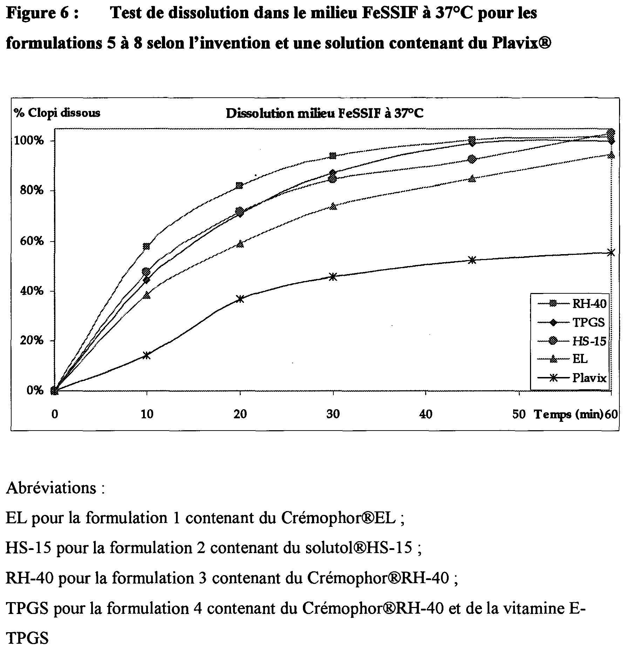

Les résultats sont présentés dans la Figure 6. On observe qu'après 1 heure moins de 56% du clopidogrel contenu dans Plavix® est dissous, alors que presque la totalité (94%) l'est pour les formulations 5 à 8.

On en conclut que la dissolution est améliorée en milieux intestinaux simulés tant à jeun que nourri.

### EXEMPLE 3 : TEST DE SOLUBILITE-

### COMPARAISON ENTRE LES FORMULATIONS SELON L'INVENTION ET LA SPECIALITE PLAVIX®

L'objectif de cet essai était de vérifier si la solubilité du clopidogrel base (qui est quasi-nulle dans l'eau) est nettement améliorée dans le cas des formulations de l'invention, et comparable à celle du Plavix® qui se trouve sous forme de sel. On effectue une test de solubilité à saturation du clopidogrel dans un milieu intestinal simulé (tel que défini dans la Pharmacopée US : *Simulated intestinal Fluid* USP XXI) à un pH de 7,5 ; à une température de 37°C. Le mode opératoire est similaire à celui de l'exemple précédent. Les résultats sont fournis dans le tableau 3 suivant. Le milieu intestinal est constitué par 6,8g de KH₂PO₄ dans 1 l d'eau et le pH ajusté à 7,5 avec KOH.

**Tableau 3 : Test de solubilité entre les formulations 5 à 8 et Plagia®**

| | Tensioactif non-ionique hydrophile | Solubilité en mg/ml |
|---|---|---|
| Formulation 5 | Crémophor®EL | 79,9 |
| Formulation 6 | Solutol®HS-15 | 72,5 |
| Formulation 7 | Crémophor®RH-40 | 84,4 |
| Formulation 8 | Crémophor®RH-40 | 83 |
| | Vitamine E/TPGS | |
| Plavix® | néant | 33,4 |

D'après ces résultats, on observe que dans le milieu intestinal (à pH 7,5) la solubilité du clopidogrel pour le Plavix® est réduite (33 mg/ml). En revanche, on constate que dans le cas des formulations 5 à 8, la solubilité du clopidogrel est grandement augmentée par rapport au Plavix® (plus de 2 fois), ce qui constitue un atout favorable pour améliorer la biodisponibilité. On note également que cette solubilité (améliorée) est sensiblement identique pour les formulations 5 à 8.

### EXEMPLE 4: MESURE DU pH - Comparaison entre les formulations selon l'invention et la spécialité Plavix®.

On a mesuré le pH pour les formulations de l'invention de façon à évaluer leur effet inducteur d'acidité en milieu aqueux en comparaison avec la forme commercialisée du clopidogrel, le Plavix®. Chacune des formulations 5 à 8 ainsi qu'un comprimé de Plavix® est dissoute dans 100 ml d'eau à 20°C. Les résultats sont présentés dans le tableau 4.

**Tableau 4 : Mesure du pH pour les formulations 5 à 8 et une solution contenant du Plavix®**

| | Tensioactif non-ionique | pH dans 100 mL d'eau à 20°C(+/- 0,1) |
|---|---|---|
| Formulation 5 | Crémophor®EL | 5,1 |
| Formulation 6 | Solutol®HS-15 | 5,2 |
| Formulation 7 | Crémophor®RH-40 | 5,3 |
| Formulation 8 | Crémophor®RH-40+ | 5,2 |
| | Vitamine E/TPGS | |
| Plavix® | néant | 2,6 |

D'après ces résultats, on constate que les formulations de l'invention induisent un pH physiologiquement plus proche de la neutralité (entre 5 et 6,1) que le Plavix® qui induit un pH très acide (inférieur à 3). Ceci constitue un avantage non négligeable pour des formulations orales.

## Revendications

1. Composition pharmaceutique orale constituée par le clopidogrel sous forme de base en tant que principe actif et au moins un tensioactif non-ionique hydrophile, cette composition ayant un pH compris entre 5 et 8 après dilution dans l'eau , ledit tensioactif non-ionique hydrophile étant choisi parmi des huiles de ricin polyoxyéthylénées, des esters de polyoxyéthylène de l'acide 12-hydroxystéarique, le mélange d'huile de ricin polyoxyéthylénée et du succinate du d-α-tocophéryl polyéthylène glycol 1000, ou bien un mélange de ceux-ci.

2. Composition pharmaceutique selon la revendication précédente, **caractérisée en ce que** le tensioactif non-ionique hydrophile est choisi parmi
une huile de ricin polyoxyéthylénée et le mélange d'une l'huile de ricin polyoxyéthylénée et du succinate du d-α-tocophéryl polyéthylène glycol 1000.

3. Composition pharmaceutique selon l'une des revendications précédentes, **caractérisée en ce qu'**en outre elle comprend un antioxydant et/ou un conservateur.

4. Composition pharmaceutique selon l'une des revendications précédentes, **caractérisée en ce que** la solubilité du clopidogrel est supérieure à 70 mg/ml dans un milieu intestinal selon la pharmacopée USP XXI à pH=7,5 à une température de 37°C.

5. Composition pharmaceutique selon l'une des revendications 1 à 4, **caractérisée en ce que** la dissolution du clopidogrel est supérieure à 95 % dans des milieux digestifs simulés FeSSIF ou FaSSIF en 30 minutes à une température de 37°C et sous agitation de 50 tours par minute.

6. Gélule contenant une composition pharmaceutique selon l'une des revendications précédentes, **caractérisée en ce qu'**elle contient du clopidogrel sous forme de base à un dosage compris entre 5 et 600 mg, la quantité de clopidogrel base étant égale à 300 mg, avantageusement à 75 mg.

7. Gélule selon la revendication 6, **caractérisée en ce que** la formulation contient, outre le clopidogrel, au moins un tensioactif non ionique hydrophile choisi parmi des huiles de ricin polyoxyéthylénées , des esters de polyoxyéthylène de l'acide 12-hydroxystéarique, une huile de ricin polyoxyéthylénée et un mélange d'huile de ricin polyoxyéthylénée et de succinate du d-α-tocophéryl polyéthylène glycol 1000 selon une quantité comprise entre 250 mg et 600 mg.

8. Gélule selon la revendication 6 ou 7, **caractérisée en ce que** la formulation contient 75 mg de clopidogrel sous forme de base et 290 mg d'huile de ricin polyoxyéthylénée ou qu'elle contient 75 mg de clopidogrel sous forme de base, 287 mg de d'huile de ricin polyoxyéthylénée et 3 mg de du succinate du d-α-tocophéryl polyéthylène glycol 1000.

9. Procédé de préparation de la composition pharmaceutique selon l'une des revendications 1 à 5, comprenant les étapes suivantes :
(a) au moins un tensioactif non-ionique hydrophile présentant une valeur de HLB> 10 est préalablement chauffé à une température comprise entre 30°C et 60°C ;
(b) le principe actif est dissous sous agitation à une température comprise entre 30°C et 60°C dans au moins un tensioactif non-ionique hydrophile préalablement chauffé(s).

10. Procédé de préparation des gélules selon l'une des revendications 6 à 8 comprenant les étapes suivantes :
(a) au moins un tensioactif non-ionique hydrophile présentant une valeur de HLB> 10 est préalablement chauffé à une température comprise entre 30°C et 60°C ;
(b) le principe actif est dissous sous agitation à une température comprise entre 30°C et 60°C dans au moins un tensioactif non-ionique hydrophile préalablement chauffé ;
(c) le mélange ainsi obtenu est éventuellement refroidi à température ambiante ;
(d) le mélange est éventuellement chauffé à une température comprise entre 30°C et 45°C ;
(e) le mélange est transféré dans les gélules.

11. Utilisation d'une composition pharmaceutique selon l'une des revendications 1 à 5, ou d'une gélule selon l'une des revendications 6 à 8, dans la préparation d'un médicament à utiliser dans la prévention de pathologies liées à l'artérothrombose,
dans la préparation d'un médicament destiné à des patients traités dans la prévention des dites pathologies liées à l'artérothrombose et qui ont auparavant subi des effets indésirables liés à l'utilisation de spécialités classiques contenant un agent antiplaquettaire,
dans la préparation d'un médicament destiné à des patients traités dans la prévention des dites pathologies liées à l'artérothrombose et qui ont auparavant subi des effets indésirables liés à l'hémolyse,
dans la préparation d'un médicament destiné à des patients traités dans la prévention des dites pathologies liées à l'artérothrombose et qui ont auparavant subi des effets indésirables liés à l'acidité de l'agent antiplaquettaire sous sa forme de sel,
dans la préparation d'un médicament destiné à des patients traités dans la prévention des dites pathologies liées à l'artérothrombose et qui sont des patients déficitaires en glucose- 6-phosphate déshydrogénase (G6PG), ou des patients souffrant d'atteintes hématologiques comme la microangiopathie thrombotique, ou alors des patients souffrant du syndrome hémolytique urémique, ou
dans la préparation d'un médicament destiné à des patients traités dans la prévention des dites pathologies liées à artérothrombose et qui sont des patients souffrant d'ulcère gastroduodénal ou de saignements gastro-intestinaux.

## Patentansprüche

1. Orale pharmazeutische Zusammensetzung, die Clopidogrel in Form der Base als Wirkstoff und wenigstens ein hydrophiles nichtionisches Tensid besteht, wobei diese Zusammensetzung nach Verdünnung in Wasser einen pH-Wert zwischen 5 und 8 aufweist, wobei das hydrophile nichtionische Tensid aus polyoxyethylenierten Ricinusölen, Polyoxyethylen-12-hydroxystearaten, dem Gemisch aus polyoxyethyleniertem Ricinusöl und α-Tocopherylpolyethylenglycol-1000-succinat, oder einem Gemisch derselben ausgewählt ist.

2. Pharmazeutische Zusammensetzung gemäß dem vorstehenden Anspruch, **dadurch gekennzeichnet, dass** das hydrophile nichtionische Tensid ausgewählt ist aus einem polyoxyethylenierten Ricinusöl und dem Gemisch aus polyoxyethyleniertem Ricinusöl und α-Tocopherylpolyethylenglycol-1000-succinat.

3. Pharmazeutische Zusammensetzung gemäß einem der vorstehenden Ansprüche, **dadurch gekennzeichnet, dass** sie weiterhin ein Antioxidans und/oder einen Konservierungsstoff umfasst.

4. Pharmazeutische Zusammensetzung gemäß einem der vorstehenden Ansprüche, **dadurch gekennzeichnet, dass** die Löslichkeit des Clopidogrels in einem Darmmilieu gemäß dem Arzneibuch USP XXI bei pH = 7,5 und einer Temperatur von 37 °C mehr als 70 mg/ml beträgt.

5. Pharmazeutische Zusammensetzung gemäß einem der Ansprüche 1 bis 4, **dadurch gekennzeichnet, dass** der Auflösungsgrad des Clopidogrels in simulierten FeSSIF- (nach dem Essen) oder FaSSIF-Verdauungsmilieus (nüchtern) in 30 Minuten bei einer Temperatur von 37 °C und unter Rühren mit 50 U/min mehr als 95% beträgt.

6. Kapsel, die eine pharmazeutische Zusammensetzung gemäß einem der vorstehenden Ansprüche enthält, **dadurch gekennzeichnet, dass** sie Clopidogrel in Form der Base in einer Dosierung zwischen 5 und 600 mg enthält, wobei die Menge der Clopidogrelbase 300 mg, vorteilhafterweise 75 mg, beträgt.

7. Kapsel gemäß Anspruch 6, **dadurch gekennzeichnet, dass** die Zubereitung außer Clopidogrel wenigstens ein hydrophiles nichtionisches Tensid, das aus polyoxyethylenierten Ricinusölen, Polyoxyethylen-12-hydroxystearaten, einem polyoxyethylenierten Ricinusöl und einem Gemisch aus polyoxyethyleniertem Ricinusöl und α-Tocopherylpolyethylenglycol-1000-succinat in einer Menge zwischen 250 mg und 600 mg ausgewählt ist, enthält.

8. Kapsel gemäß Anspruch 6 oder 7, **dadurch gekennzeichnet, dass** die Zubereitung 75 mg Clopidogrel in Form der Base und 290 mg polyoxyethyleniertes Ricinusöl enthält oder dass sie 75 mg Clopidogrel in Form der Base, 287 mg polyoxyethyleniertes Ricinusöl und 3 mg α-Tocopherylpolyethylenglycol-1000-succinat enthält.

9. Verfahren zur Herstellung der pharmazeutischen Zusammensetzung gemäß einem der Ansprüche 1 bis 5, umfassend die folgenden Schritte:
(a) Vorwärmen wenigstens eines hydrophilen nichtionischen Tensids, das einen HLB-Wert > 10 aufweist, auf eine Temperatur zwischen 30 °C und 60 °C;
(b) Auflösen des Wirkstoffs unter Rühren bei einer Temperatur zwischen 30 °C und 60 °C in wenigstens einem vorgewärmten hydrophilen nichtionischen Tensid.

10. Verfahren zur Herstellung von Kapseln gemäß einem der Ansprüche 6 bis 8, umfassend die folgenden Schritte:
(a) Vorwärmen wenigstens eines hydrophilen nichtionischen Tensids, das einen HLB-Wert > 10 aufweist, auf eine Temperatur zwischen 30 °C und 60 °C;
(b) Auflösen des Wirkstoffs unter Rühren bei einer Temperatur zwischen 30 °C und 60 °C in wenigstens einem vorgewärmten hydrophilen nichtionischen Tensid;
(c) gegebenenfalls Abkühlen des so erhaltenen Gemischs auf Umgebungstemperatur;
(d) gegebenenfalls Erwärmen des Gemischs auf eine Temperatur zwischen 30 °C und 45 °C;
(e) Überführen des Gemischs in die Kapselhüllen.

11. Verwendung einer pharmazeutischen Zusammensetzung gemäß einem der Ansprüche 1 bis 5 oder einer Kapsel gemäß einem der Ansprüche 6 bis 8
bei der Herstellung eines Medikaments zur Verwendung bei der Prävention von Erkrankungen, die mit arterieller Thrombose zusammenhängen;
bei der Herstellung eines Medikaments für Patienten, die in der Prävention der Erkrankungen, die mit arterieller Thrombose zusammenhängen, behandelt werden und die zuvor Nebenwirkungen erlitten haben, die mit der Verwendung von klassischen Präparaten, die einen Thrombozytenaggregationshemmer enthalten, zusammenhängen;
bei der Herstellung eines Medikaments für Patienten, die in der Prävention der Erkrankungen, die mit arterieller Thrombose zusammenhängen, behandelt werden und die zuvor Nebenwirkungen erlitten haben, die mit einer Hämolyse zusammenhängen;
bei der Herstellung eines Medikaments für Patienten, die in der Prävention der Erkrankungen, die mit arterieller Thrombose zusammenhängen, behandelt werden und die zuvor Nebenwirkungen erlitten haben, die mit der Acidität des Thrombozytenaggregationshemmers in seiner Salzform zusammenhängen;
bei der Herstellung eines Medikaments für Patienten, die in der Prävention der Erkrankungen, die mit arterieller Thrombose zusammenhängen, behandelt werden und bei denen es sich um Patienten, die einen Mangel an Glucose-6-phosphat-Dehydrogenase (G6PG) aufweisen, oder um Patienten, die an einer hämatologischen Störung, wie thrombotische Mikroangiopathie, leiden, oder auch um Patienten, die an hämolytisch-urämischem Syndrom leiden, handelt; oder
bei der Herstellung eines Medikaments für Patienten, die in der Prävention der Erkrankungen, die mit arterieller Thrombose zusammenhängen, behandelt werden und bei denen es sich um Patienten handelt, die an gastroduodenalem Ulkus oder Magen-Darm-Blutungen leiden.

## Claims

1. Oral pharmaceutical composition constituted by the clopidogrel in the form of free base as active ingredient and at least one hydrophilic non-ionic surfactant, said composition having a pH comprised between 5 and 8 after dilution in water, said hydrophilic non-ionic surfactant being selected from polyoxyethylated castor oils, polyoxyethylene esters of 12-hydroxystearic acid, the mixture of polyoxyethylated castor oil and d-α-tocopherol polyethylene glycol 1000 succinate, or a mixture thereof.

2. Pharmaceutical composition according to the preceding claim, **characterized in that** the hydrophilic non-ionic surfactant is selected from a polyoxyethylated castor oil and the mixture of a polyoxyethylated castor oil and d-α-tocopherol polyethylene glycol 1000 succinate.

3. Pharmaceutical composition according to one of the preceding claims, **characterized in that** furthermore it comprises an antioxidant and/or a preservative.

4. Pharmaceutical composition according to one of the preceding claims, **characterized in that** the solubility of the clopidogrel is greater than 70 mg/ml in intestinal media according to the pharmacopoeia USP XXI at pH = 7.5 at a temperature of 37°C.

5. Pharmaceutical composition according to one of claims 1 to 4, **characterized in that** the dissolution of the clopidogrel is greater than 95 % in simulated digestive media FeSSEF or FaSSEF in 30 minutes at a temperature of 37°C and under agitation of 50 revolutions per minute.

6. Capsule containing a pharmaceutical composition according to one of the preceding claims, **characterized in that** it contains the clopidogrel in the form of free base at a dosage comprised between 5 and 600 mg, the amount of clopidogrel free base being equal to 300 mg, advantageously to 75 mg.

7. Capsule according to claim 6, **characterized in that** the formulation contains, in addition to the clopidogrel, at least one hydrophilic non-ionic surfactant selected from polyoxyethylated castor oils, polyoxyethylene esters of 12-hydroxystearic acid, a polyoxyethylated castor oil and a mixture of polyoxyethylated castor oil and d-α-tocopherol polyethylene glycol 1000 succinate in an amount comprised between 250 mg and 600 mg.

8. Capsule according to one of claims 6 or 7, **characterized in that** the formulation contains 75 mg of the clopidogrel in the form of free base and 290 mg of polyoxyethylated castor oil or it contains 75 mg of the clopidogrel in the form of free base, 287 mg of polyoxyethylated castor oil and 3 mg of d-α-tocopherol polyethylene glycol 1000 succinate.

9. Method of preparing the pharmaceutical composition according to one of claims 1 to 5, comprising the following steps:
(a) at least one hydrophilic non-ionic surfactant presenting a value of HLB >10 is initially heated to a temperature comprised between 30°C and 60°C;
(b) the active ingredient is dissolved under agitation at a temperature comprised between 30°C and 60°C in at least one non-ionic hydrophilic surfactant previously heated.

10. Method of preparing the capsules according to one of claims 6 to 8, comprising the following steps:
(a) at least one hydrophilic non-ionic surfactant presenting a value of HLB >10 is initially heated to a temperature between 30°C and 60° C;
(b) the active ingredient is dissolved under agitation at a temperature comprised between 30°C and 60°C in at least one non-ionic hydrophilic surfactant previously heated;
(c) the mixture thus obtained is optionally cooled to room temperature;
(d) the mixture is optionally heated to a temperature comprised between 30°C and 45°C;
(e) the mixture is transferred into the capsules.

11. Use of a pharmaceutical composition according to one of claims 1 to 5, or a capsule according to one of claims 6 to 8, in the preparation of a medicament for use in the prevention of pathologies linked to the arterothrombose,
in the preparation of a medicament for patients treated in the prevention of the pathologies linked to arterothrombose and which have previously undergone undesirable effects associated with the use conventional specialties containing an antiplatelet agent,
in the preparation of a medicament for patients treated in the prevention of the pathologies linked to the arterothrombose and which have previously undergone undesirable effects associated with hemolysis,
in the preparation of a medicament for patients treated in the prevention of the pathologies linked to the arterothrombose and have previously undergone of adverse effects linked to the acidity of the anti-platelet agent in the form of its salt,
in the preparation of a medicament for patients treated in the prevention of the pathologies linked to the arterothrombose and which are patients deficient for glucose-6-phosphate dehydrogenase (G6PG), or patients suffering from hematological damage such as thrombotic microangiopathy, or patients with uremic haemolytic syndrome,
in the preparation of a medicament for patients treated in the prevention of the pathologies linked to arterothrombose and which are patients with peptic ulcer or gastrointestinal bleeding.
